# EUROPEAN PATENT SPECIFICATION

(11) **EP 0 913 397 B1**
(45) Date of publication and mention of the grant of the patent: **05.07.2006**
(21) Application number: 97308381.9
(22) Date of filing: 22.10.1997
(51) Int. Cl.: C07D 403/06, A61K 31/495

(54) **A proces for the synthesis of 1-(4-Arylpiperazine-1-y1)-3-(2-oxypyrrolidin/piperidin-1-yl)propanes as therapeutic agents for hypertension, ischemia, cardiovascular and other adrenergic receptors related disorders**
Verfahren zur Synthese von 1-(4-Arylpiperazin-1-yl)-3-(oxopyrrolidin/piperidin-1-yl)propanen als therapeutische Mittel bei Bluthochdruck, Ischämie, kardiovaskulären und anderen, mit adrenergenen Rezeptoren im Zusammenhang stehenden Störungen
Procédé de synthèse de 1-(4-arylpipérazin-1-yl)-3-(2-oxopyrrolidin/pipéridin-1-yl)propanes comme agents thérapeutiques pour l'hypertension, l'ischémie, des troubles cardiovasculaires et d'autres troubles liés aux récepteurs adrénergiques

(43) Date of publication of application: 06.05.1999
(73) Proprietor: COUNCIL OF SCIENTIFIC AND INDUSTRIAL RESEARCH, New Delhi 110 001 (IN)
(72) Inventor: Sinha, Neelima, Chattar Manzil Palace Lucknow 226 001,UP (IN); Jain, Sanjay, Chattar Manzil Palace Lucknow 226 001,UP (IN); Saxena, Anil Kumar, Chattar Manzil Palace Lucknow 226 001,UP (IN); Anand, Nitya, Chattar Manzil Palace Lucknow 226 001,UP (IN); Saxena, Ram Mohan, Chattar Manzil Palace Lucknow 226 001,UP (IN); Dubey, Mangal Prasad, Chattar Manzil Palace Lucknow 226 001,UP (IN); Patnaik, Gyanendra Kumar, Chattar Manzil Palace Lucknow 226 001,UP (IN)
(74) Representative: Wain, Christopher Paul

(56) References cited:
- GB-A- 2 023 594
- CHEMICAL ABSTRACTS, vol. 100, no. 7, 13 February 1984 Columbus, Ohio, US; abstract no. 44907p, B. MALAWSKA ET AL.: "Synthesis and pharmacological properties of some 2-pyrrolidinone Mannich bases" page 17; XP002056654 & POL. J. PHARMACOL. PHARM., vol. 34, no. 5-6, 1982, pages 373-382,
- P. E. CROSS ET AL.: "Substituted trifluoromethyl phenyl piperazines as anorectic agents" EUR. J. MED. CHEM. - CHIM. THER., vol. 12, no. 2, 1977, pages 173-176, XP002056653

## Description

### FIELD OF THE INVENTION

The present invention relates, to a process for the synthesis of novel 1-[4-Arylpiperazin-1-yl]-3-[2-oxopyrrolidin-1-yl]propanes and 1-[4-Arylpiperazin-1-yl]-3-[2-oxopiperidin-1-yl]propanes which can be used as therapeutic agents for hypertension, ischemia, cardiovascular and other adrenergic receptors related disorders. More, particularly the present invention relates, to a process for the synthesis of 1-[4-[Arylpiperazin-1-yl]-3-[2-oxopyrrolidin-1-yl]propanes and 1-[4-Arylpiperazin-1-yl]-3-[2-oxopiperidin-1-yl]propanes, and to their use in medicine. This invention provides the compounds of the formula 1.

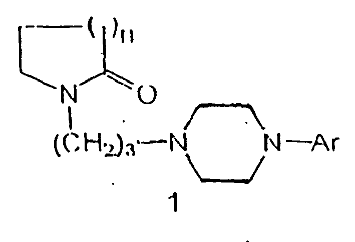

Wherein Ar represents a phenyl ring substituted by the groups like halo, alkoxy, alkyl or heteroaryl and n=1 or n=2.

The compounds of the invention have shown to possess antihypertensive activity in different test models. The compounds also prevent post-ischemic reperfusion injury and may be useful in the treatment of hypertension, diseases arising out of alterations/impairment in central/peripheral circulations and adrenergic receptors systems such as myocardial ischemia, myocardial infarction (MI), angina pectoris, any cardiac surgical interventions renal ischemia, circulatory insufficiency in extremities, stroke and trauma.

A general method of preparation of the inventive compounds starts from the condensation of 1-bromo-3-chloropropane with 2-pyrrolidone or 2-piperidone to give the key intermediate 1-chloro-3-[2-oxopyrrolindin-1-yl]propanes (n=1) of formula 3 or 1-chloro-3-[2-oxopiperidin-1-yl]propanes (n=2) followed by its condensation with different 1-substituted piperazines of formula 4 to get the compounds of formula 1 and the said method is the subject matter of the co-pending US application number ............. According to the method, the process of which starts from the condensation of 1-bromo-3-chloropropane with different 1-substituted piperazines of formula 4 to give the 1-chloro-3-(4-substituted piperazin-1-yl)propanes of formula 5 followed by their condensation with 2-pyrrolidone or 2-piperidone of formula 2 to get the compounds of formula 1 (formula 2 to 5 are shown in scheme 1 of the accompanying drawings).

The compounds of the present invention can be used as pharmaceutical compositions comprising compounds of the present invention with a suitable pharmaceutical carrier. Preferably, these compositions are used to produce antihypertensive and antiischemic activities and contain an effective amount of the compounds useful in the method of the invention. The most preferred compound of the invention is 1-[4-(4-fluorophenyl)piperazin-1-yl]-3-[2-oxopyrrolidin-1-yl]propane.

### BACKGROUND OF THE INVENTION

Hypertension is the most common of all cardiovascular diseases afflicting about 10-20% adult population. Several classes of drugs may be used in the treatment and management of hypertension such as alpha-adrenoceptor antagonists, ACE inhibitors, angiotensin I chymase inhibitors, renin inhibitors, angiotensin II antagonists, vasopressin V₁ antagonists, endothelin antagonists, endothelin-converting enzyme inhibitors, potassium channel activators, calcium channels antagonists, adenosine A₂ agonists, adenosine A₁ antagonists, neutral endopeptidase inhibitors, dual-action ACE and neutral endopeptidase inhibitors.

These drugs belong to structurally diverse class of heterocyclics including substituted arylpiperazines. In this context, the 1-[4-Arylpiperazin-1-yl]-3-[2-oxopyrrolidin-1-yl]propanes and 1-[4-Arylpiperazin-1-yl]-3-[2-oxopiperidin-1-yl]propanes of the formula 1 are structurally novel compounds and show significant antihypertensive and antiischemic activities. Thus, these compounds would be useful in the treatment of hypertension and in preventing post-ischemic reperfusion injury (ischemia).

The most commonly used antihypertensive drugs are ACE's inhibitors (captopril and related drugs), Ca⁺⁺ channel blocker (nifedipine, verapamil, diltiazen) and peripheral alpha₁- adrenergic antagonist such as prazosin. As these drugs have one or the other side effects, there has been a continuous search for new and safe antihypertensive agents acting by these mechanism and by other novel mechanism which include mainly endothelin antagonists [Gulati, A. and Srimal, R.C. *Drug Dev. Res., 26,* 361, 1992; Antihypertensive Drugs. *The Year's Drug News,* 145-167, 1994].

There are no drugs available to prevent post-ischemic reperfusion injury. However, the existing drugs or chemical agents like Ca⁺⁺ channel blockers [Hensch, G. *Cardiovascular Res.,* 26, 14, 1992; Karin Pazyklenk, Robert A. Kloner. *Cardiovascular Research,* 26 82, 1992], K_{ATP} openers [Allen W. Gomoll et al., *J. Phalmacol. Exp. Ther.,* 281, 24, 1997; Arthur A.M. Wilde, *Cardiovascular Research,* 35, 181, 1997] Na⁺/H⁺ exchange inhibitors [Wolfgang Scholz *et al., Cardiovas. Res*., 29, 260, 1995] have been shown to promote myocardial salvage and enhance function recovery *in vivo,* only when given before or during ischemic episode. However, administration of these agents only during reperfusion does not result in cardioprotective activity (Grover, G.J. *et al., Cardiovasc. Drugs Ther.,* 4, 465, 1990 & *Eur. J. Pharmacol*., 191, 11, 1990; Mizumura, T. *et al., Circulation*, 92, 1236, 1995). Besides the use of antiischemic agents in prevention of ischemic/reperfusion injury, there is an unmet medical need for agents to treat post-ischemic reperfusion injury which may simulate the real clinical situation of myocardial infarction.

### PRIOR ART

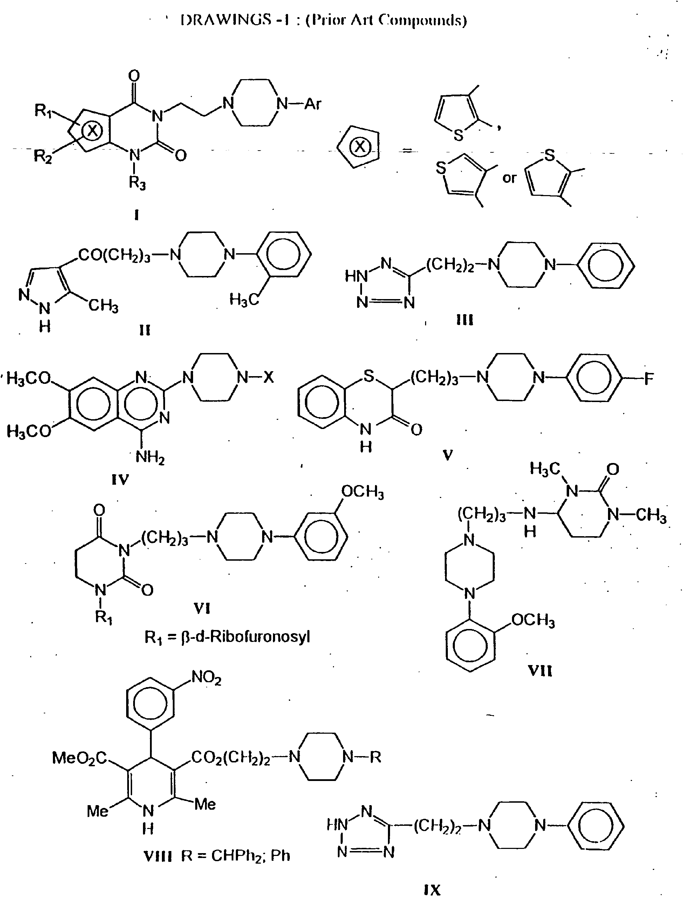

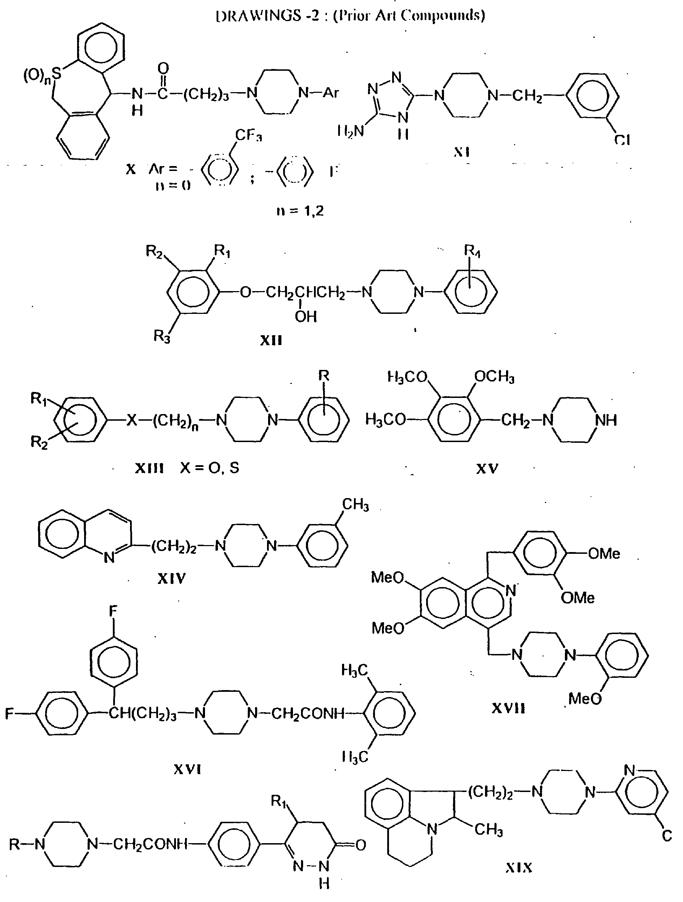

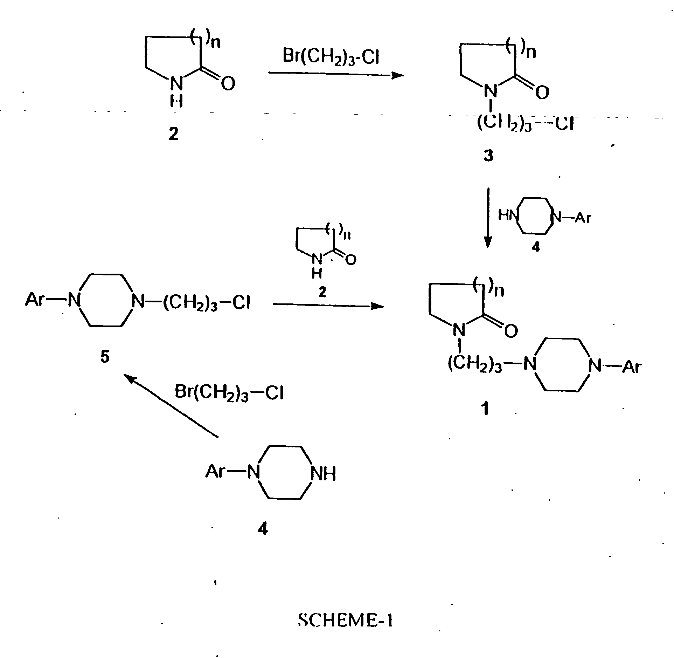

Among a large number of the molecules incorporating arylpiperazines and showing antihypertensive activity, some relevant ones are thienopyrimidine-2,4-diones of formula I of the accompanying drawings - 1 (US Patent No. 4,670560, 1989), pyrazoles of formula II of the accompanying drawings - 1 (Arya, V.P. *et al., Experentia,* 23, 514, 1967), tetrazoles of formula III of the accompanying drawings - I (Hayae, S. *et al. J. Med. Chem.,* 10, 400, 1967), prazocin analogs of formula IV of the accompanying drawings -1 (Luther, R.R. *et al., Am. Heart J*., 117, 842, 1989; Ames, R.P. & Kiyasu, J.Y. *J. Clin. Pharmacol.,* 29, 123, 1989), 2-[3-[4-(4-fluorophenyl)piperazin-1-yl]propanes]-1,4-benzothiazin-3(4H)-one of formula V of the accompanying drawings - 1 (Kajine, M. *et al., Chem. Pharm. Bull.,* 39, 2885, 1991), uracil derivatives of formula VI & VII of the accompanying drawings - 1 (Klmm, Von K. *et al., Arzneim. Forsch.,* 27, 1875, 1977), dihydropyridines of formula VIII of the accompanying drawings 1 (Suzuki H. & Saruta, T, *Cardiovasc. Drug Rev.,* 7, 25, 1989; Kubo, K. and Karawasawa, A. *10th Int. Cong. Pharmacol*., 734, 1987; *Drugs of the Future,* 14, 291, 1989; Meguro, K. *et al., Chem. Pharm. Bull*., 33, 3787, 1985; Nakaya, H. *et al., Eur. J. Pharmacol*., 146, 35, 1988; Kakihand, M. *et al., Jpn. J. Pharmacol*., 48, 223, 1988; Takenaka, T. *et al., Arzneim. Forsch.,* 26, 2127, 1976; Kajimo, M. *et al., Chem. Pharm. Bull*., 37, 2225, 1989; Tricerri, S.Z. *et al*., US Pat. 4,894,460, 1990: *Chem. Abst*., 113, 132218b, 1990), zolertine of formula IX of the accompanying drawings - 1 (Arya, V.P. *et al., Experientia*, 23, 514, 1967; Hayao, S. *et al., J. Med. Chem*., 10, 400, 1967), thiepin derivatives of formula X of the accompanying drawings - 2 (Uno, H. *et al*., US Patent. 4,749,703, 1988), triazolylamine of formula XI of the accompanying drawings -1 (Mayer, W.E. *et al., J. Med. Chem*., 32, 593, 1989), aryloxypropanolamines of formula XII of the accompanying drawings - 2 (Ing, H.R. & Ormerod, W.E., *J. Pharm. Pharmacol.,* 4, 21, 1952; Petrow, V. *et al., J. Pharm. Pharmacol*., 8, 666, 1956; Moran, N.C. and Perkins, M.E., *Pharmacol. Exp. Ther.,* 124, 223, 1958), aryloxy/thioaryloxy arylpiperazinylpropanes of formula XIII of the accompanying drawings - 2 (Agarwal, S.K. *et al., Ind. J. Chem*., 2 1 B, 435, 1982; *Ind. J. Chem.,* 21B, 914, 1982; *Ind. J. Chem*., 30B, 413, 1991; Rao, J. *et al., Ind. J. Chem.,* 26B, 761, 1987; Saxena, A.K. *et al., Ind. J. Chem*., 32B, 1249, 1993), quinolylethanes of formula XIV of the accompanying drawings - 2 Murti, A. *et al., Ind. J. Chem.,* 28B, 934, 1989), trinetazidine of formula XV of the drawing 2 (Fujita, Y, *Jpn. J. Pharmacol.,* 17, 19, 1976), lidoflazine of formula XVI of the drawing 2 (Daenen, W. & Flameng, W., *Angiology,* 32, 543, 1981), isoquinolylmethyl derivatives of formula XVII of the accompanying drawings - 2 (Nakajiza, T. *et al., Arzneim-Forsch.,* 37, 674, 1987), dihydropyridazinone derivative of formula XVIII of the accompanying drawings - 2 (Yao, F.M. *et al*., *Yaaxue Xuebao*, 28, 548, 1993), pyrroloquinoline derivative of formula XIX of the accompanying drawings - 2 (Jasserand, D. *et al.,* Ger. Offen. DE 4,128,015:, 1993 *Chem. Abst*., 119, 139255f, 1993).

### DETAILED DESCRIPTION OF THE INVENTION

The invention is mainly centered around the following objects:
(i) The first object of the invention is to provide a Process for preparing novel molecules incorporating piperazine flanked on one side by aromatic system and on the other side by 2-(oxopyrrolidin-1-yl)propanes or 2-(oxopiperidin-1-yl)propanes that exhibit better therapeutic efficacy to treat hypertension over the existing antihypertensive agents.
(ii) The second object of the invention is to provide a process for preparing novel 1-(4-arylpiperazin-1-yl)-3-(2-oxopytrrolidin-1-yl) propanes and 1-(4-arylpiperazin-1-yl)-3-(2-oxopiperidin-1-yl) propanes exhibiting activity against ischemic reperfusion injury for which there is no agent available till date to best of our knowledge.
(iii) The third object of the invention is to provide 1-(4-arylpiperazin-1-yl)-3-(2-oxopyrrolidin-1-yl) propanes and 1-4-arylpiperazin-1-yl)-3-2-oxopiperidin-1-yl) propanes as the therapeutic agents for the diseases arising out of alterations/impairment in central/peripheral circulations and adrenergic receptors systems, such as myocardial ischemia, myocardial infarction (MI), angina pectoris, any cardiac surgical interventions, renal ischemia, circulatory insufficiency in extremities, stroke and trauma.

This invention is concerned with novel pharmacologically active substances and relates to new 1-(4-arylpiperazin-1-yl)-3-(2-oxopyrrolidin-1-yl) propanes and 1-(4-arylpiperazin-1-yl)-3-(2-oxopyrrolidin-1-yl) propanes as potential therapeutic agents for hypertension, ischemia, cardiovascular and other adrenergic receptors related disorders.

According to the present invention, there is provided a process for preparing compounds of formula I 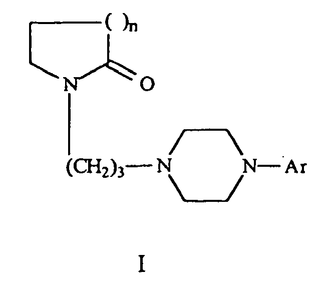 wherein Ar represents pyridyl or phenyl or a phenyl group substituted by one or more alkyl, alkoxy or halogen groups, or by one or more CF₃ groups, n=1 or n=2; which process comprises condensing 2-pyrrolidone of formula 2 (n=1) or 2-piperidone of formula 2 (n=2) with 1-[4-substituted arylpiperazin-1-yl]-3-chloropropanes of formula 5 where Ar is above in the presence of a base and organic solvent at a temperature ranging from 120-150°C for a period varying between 90 min. to 14 hrs. to produce the corresponding 1-[4-substituted arylpiperazin-1-yl]-3-[2-oxopyrrolidin/piperazin-1-yl] propanes of formula I.

The invention also provides compounds of formula I as defined above, and their use as medicaments.

In a further aspect, there is provided the use of a compound of the invention in the manufacture of a medicament for treating any of the following disorders: hypertension in mammals, peripheral vascular diseases in mammals, diseases arising out of alterations/impairment in central/peripheral circulations and adrenergic receptors systems, such as myocardial ischemia, myocardial infarction (MI), angina pectoris, any cardiac surgical interventions, renal ischemia, circulatory insufficiency in extremities, stroke and trauma, reperfusion injury in mammals, ischemic diseases like myocardial infarction (MI) or angina pectoris.

The preferred compounds of formula I are shown here below:
(a) 1-[4-(3-chlorophenyl)piperazin-1-yl]-3-[2-oxopyrrolidin-1-yl] propane.
(b) 1-[4-(4-chlorophenyl)piperazin-1-yl]-3-[2-oxopyrrolidin-I-yl] propane.
(c) 1-[4-(3-fluorophenyl)piperazin-1-yl]-3-[2-oxopyrrolidin-1-yl] propane.
(d) 1-[4-(4-fluorophenyl)piperazin-1-yl]-3-[2-oxopyrrolidin-1-yl] propane.
(e) 1-[4-(4-ethylphenyl)piperazin-1-yl]-3-[2-oxopyrrolidin-1-yl] propane.
(f) 1-[4-(2-ethylphenyl)piperazin-1-yl]-3-[2-oxopyrrolidin-1-yl] propane.
(g) 1-[4-(4-chlorophenyl)piperazin-1-yl]-3-[2-oxopiperidin-1-yl] propane.
(h) 1-[4-(3-chlorophenyl)piperazin-1-yl]-3-[2-oxopiperidin-1-yl] propane.
(i) 1-[4-(4-fluorophenyl)piperazin-1-yl]-3-[2-oxopiperidin-1-yl] propane.
(j) 1-[4-(2-ethylphenyll)piperazin-1-yl]-3-[2-oxopiperidin-1-yl] propane.
(k) 1-[4-(2-methoxyphenyl)piperazin-1-yl]-3-[2-oxopiperidin-1-yl] propane.
(l) 1-[4-(2-pyridyl)piperazin-1-yl]-3-[2-oxopiperadin-1-yl]propane.
(m) 1-[4-(3-trifluoromethylphenyl)piperazin-1-yl]-3-[2-oxopiperadinl-yl] propane.

In the specification and claims, the compounds with n=1 designates 2-oxopyrrolidin-1-yl while with n=2, 2-oxopiperidin-1-yl groups. Aryl designates a pyridyl or phenyl, or a phenyl group substituted by one or more alkyl, alkoxy or halogen groups. A preferred group of compound comprises those in which n=1 or n=2, aryl group is 2 or 4-pyridyl, phenyl, or phenyl group substituted by groups like H or CF₃, alkyl groups like C₂H₅, alkoxy like methoxy, halo like chloro, fluoro etc. The compounds of this invention have useful biological activities and have in particular strong antihypertensive and antiischemic activities.

### DESCRIPTION OF PREFERRED EMBODIMENTS

The general reaction sequence leading to 1-[4-arylpiperazin-1-yl]-3-[2-oxopyrrolidin-1-yl] propanes or 1-4-arylpiperazin-1-yl]-3-2-oxopiperidin-1-yl] propanes is shown in scheme 1 of the accompanying drawings.

It will be noted that according to the foregoing schemes there are two general methods leading to the synthesis of 1. In the first general method the 2-pyrrolidone (2, n=1) or 2-piperidone (2, n=2) is condensed with 1-bromo-3-chloropropane in presence of bases selected from potassium *tert.* butoxide, pulverized alkali metals selected from sodium or potassium in nonpolar solvents selected from benzene, xylene, toluene at a temperature ranging from 110 to 150°C for 1.15 to 14 hrs to give 1-chloro-3[2-oxo-pyrrolidin-1-yl]propane (3, n=1) or 1-chloro-3-[2-oxo-piperidin-1-yl]propane (3, n=2) which on condensation with appropriately substituted piperazines, gave the required compounds of formula 1. This reaction may be carried out in solvents selected from acetone, methylethyl ketone, tetrahydrofuran or dimethylformamide using bases selected from triethylamine, pyridine, sodium or potassium carbonate and catalysts selected from sodium/potassium iodide to improve the yield of the compounds of formula 1 and the said method is the subject matter of the co-pending US application number ..........

According to the method, the substituted piperazine (4) was condensed with 1-bromo-3-chloropropane in presence of bases selected from sodium or potassium carbonate and catalytic amounts of sodium or potassium iodide in solvents selected from DMF, toluene, xylene etc. at a temperature ranging from 70 to 150°C for 8 to 14 hrs to give 1-chloro-3-(4-substituted piperazin-1-yl)propane (5) which on condensation with 2-oxo-pyrrolidine or 2-oxopiperidine in presence of bases selected from potassium *tert*. butoxide or pulverised sodium or potassium in nonpolar solvents selected from xylene, toluene at a temperature ranging from 110 to 150°C for 1.15 to 14 hrs yield the required compounds of formula 1.

The 1-[4-arylpiperazin-1-yl]-3-[2-oxopyrrolidin-1-yl]propanes (1 n=1) and 1-[4-arylpiperazin-1-yl]-3-[2-oxopiperidin-1-yl]propanes (1 n=2) in free form can, if desired be converted in to, their non-toxic pharmaceutically acceptable acid addition and quaternary ammonium salts. Salts which may be formed comprise, for example, salts with inorganic acids such as hydrochloride, hydrobromide, hydroiodide, sulphate and phosphate. They may also comprise salts with organic acids including mono basic acids such as acetate or propionate and especially those with hydroxy organic acids and dibasic acids such as the citrate, tartarate, malate and maleate. Among useful quaternary ammonium salts are those formed by such alkyl halides as methyl iodine and n-hexyl bromide.

The compounds of the invention show marked antihypertensive alpha-adrenergic blocking and antiischemic activities and can be used as therapeutic agents in diseases arising out of alterations/impairment in central/peripheral circulations and adrenergic receptors systems, such as myocardial ischemia, myocardial infarction (MI), angina pectoris, any cardiac surgical interventions, renal ischemia, circulatory insufficiency in extremities, stroke and trauma as shown for instance by the following data of the compound 1-[4-(4-fluorophenyl)piperazin-1-yl]-3-[2-oxopyrrolidin -1-yl]propane.

### Pharmacological activity

### 1. Acute toxicity (LD₅₀)

Mice 147.0 mg/kg i.p. (C.L. = 85.3-253)
562.0 mg/kg p.o. (C.L. = 383-825)

2. Effect on blood pressure, heart rate and adrenaline vasopressor response of anaesthetized (pentobarbitone sodium 40 & 60 mg/kg i.p. in cat and rat respectively) normotensive & hypertensive rat & cat model preparations.

| Dose (uMol/kg i.d.) | B.P. fall (%) | Dur. (min.) | Heart rate Pre/post treatment | Adrenaline vasopressure response % inhibition | No. of exp. |
|---|---|---|---|---|---|
| **(A) CAT** | | | | | |
| (i) Naturally occurring hypertensive cat | | | | | |
| 10 | 16 110 | | 195/195 | 49 | n=4 |
| 20 | 22 >156.75 | | 177/185 | R 26 | n=4 |

| (ii) Normotensive cat | | | | | |
|---|---|---|---|---|---|
| 10 | 22 | 85 | 200/170 | 26 | n=4 |
| 20 | 21 | >102.5 | 185/165 | 34.75 | n=4 |

| Dose (uMol/kg i.d.) | B.P. fall (%) | Dur. (min.) | Heart rate Pre/post treatment | Adrenaline vasopressure response % inhibition | No. of exp. |
|---|---|---|---|---|---|
| **(B) RAT** | | | | | |
| (i) Hypertensive rat model preparation | | | | | |
| 5 | 27 | 51 | 346/320 | 33 | n=3 |
| 10 | 22 | 76 | 356/340 | 23 | n=5 |
| 20 | 29 | 100 | 310/275 | 54 | n=1 |

| (ii) Normotensive rat model preparation | | | | | |
|---|---|---|---|---|---|
| 2 i.v. | 25 | 11 | 350/370 | +7 R22 | n=2 |
| 10 i.v. | 10 | 3 | 315/360 | +14 R28 | n=2 |
| 20 i.v. | 21 | 27.5 | 315/285 | -9.5 R42 | n=2 |

| | | | | | |
|---|---|---|---|---|---|
| i.d. = Intraduodenal route; R = Reversal | | | | | |

### 3. Possible site and mechanism of action

### (I) SITE

(i) Spinal transected cat

| Dose (uMol/kg i.d. | B.P. fall (%) | Dur. (min.) |
|---|---|---|
| 2-10 | 14-18 | 15-30 |
| 0.34-1.36 | 8-10 | 10-15 |

(ii) ICV
(iii) Rat hind limb perfusion

| Total dose (ug) | Percent change in flow |
|---|---|
| 10 | no effect |
| 25 | +35 (Vasodilatation) |
| 50 | +50 (Vasolidatation) |

### (II) MECHANISM OF ACTION

(A) *In vitro*
   (i) Isolated aortic strip:
      * Endothelin induced contraction was inhibited significantly.
      * Even after washing the preparation endothelin caused relaxation rather than contraction.
         (II) Isolated Guinea pig ileum preparation endothelin relaxation rather than contraction.
      * Compound showed significant antihistaminic activity (0.5-5.0 ug/ml).
   (iii) Langendorffs perfused rat heart preparation:
      * Lower dose of this compound (1 ug) showed some negative chronotropic effect (30% for 10 min.) but higher doses (3-5 ug) showed less negative chronotropic effect (26 & 5% for 14 & 5 min. respectively).
   (iv) Konzett and Rossler preparation:
      * Compound showed some antihistaminic activity against histamine induced bronchoconstriction.
   (v) Rat aortic ring preparation:
      * NE induced contraction was inhibited by the compound.
(B) *In vivo*
   Drug antagonism studies at 2 uMol dose i.v. in cat:
   (i) Pretreatment with alpha₁-adrenergic receptor blocker, prazosin significantly (90%) reduced antihypertensive effect.
   (ii) Pretreatment with Ca⁺⁺ channel blocker, verapamil significantly reduced antihypertensive effect (50%).
   iii) Pretreatment with captopril (an ACE inhibitor) or Dup-753 (an angiotension II-receptor antagonist) also reduced the antihypertensive effect (33%).
   (iv) ATP sensitive potassium channel (K_{ATP}) blocker glibenclamide pretreatment only partially reduced the fall in blood pressure.
   (v) Pretreatment with, atropine sulphate, mepyramine maleate, propranolol, or yohimbine failed to alter the antihypertensive effect of this compound.

### (4) Comparative antihypertensive effect with clinically used antihypertensive drugs at 2 uMol/kg i.v. dose in cat

| Drug | B.P. fall (%) | Dur. (min.) |
|---|---|---|
| (i) Verapamil | 55 | 22 |
| (ii) Captopril | 14 | 28 |
| (iii) Compound 1 of formula 1, where Ar=C₆H₄-4-F, n=1 | 22 | 25 |

### (5) Cardioprotective activity

The most interesting observation is its cardioprotective effect against myocardial stunning at a much smaller dose than antihypertensive dose. Further, in Langendorff perfused rat heart preparation subjected to even up to 90 min. global ischemia, the compound at 0.001 ug/ml conc. given at the time of reperfusion revived normal rhythmic contraction started within 2 min. (Table 1) and incidence of reperfusion induced arrhythmia were abolished.

**Table 1: Compound administered at the time reperfusion at the dose of 0.001 ug/ml on prolong period (90min.) of ischemic insult.**

| S. No. | Compound | Onset (min.) | Percentage recovery | |
|---|---|---|---|---|
| | | | 15 min. | 30 min. |
| 1. | Control^{a} | 3 | 0.5 | 17.5 |
| 2. | Compound 1 of formula 1, where Ar=C₆H₄-4-F, n=1 | 4 | 81.25 | 87.15 |
| 3. | Nifedipine^{a} (10⁻⁶M) | 2.5 | 0.66 | 0.0 |

| | | | | |
|---|---|---|---|---|
| a - Ischemic insult (45 min.) | | | | |

Comparable results for hypotensive/antihypertensive and antiischemic activities were obtained with a number of other compounds of formula I (Table 2 & 3 ).

**Table 2: Effect on blood pressure, heart rate and adrenaline vasopressure response at an anaesthetized (pentobarbitone sodium 35 mg/kg i.v.) cat.**

| Compound of formula 1 | | Dose (umol/kg i.v.) | B. P. fall % | Dur. (min.) | Adrenaline^{a} vasopressure response % inhibition |
|---|---|---|---|---|---|
| Ar | n | | | | |
| C₆H₄-4-F | 1 | 2.0 | 25 | 71 | 9 |
| | | 10.0 | 31 | >112 | R |
| C₆H₄-2-C₂H₅ | 1 | 2.0 | 13 | 10 | Pt |
| | | 10.0 | 62 | 10 | - |
| C₆H₄-3-Cl | 1 | 2.0 | 25 | 20 | Pt |
| | | 10.0 | 55 | >57 | - |
| C₆H₄-4-C₂H₅ | 1 | 2.0 | 9 | Tr | - |
| | | 10.0 | 20 | 14 | - |
| C₆H₄-3-F | 1 | 2.0 | 18 | 3 | 80 |
| | | 10.0 | 32 | 30 | R |
| C₆H₄-2-OCH₃ | 1 | 2.0 | 19 | 19 | 47 |
| | | 10.0 | 23 | 67 | R |
| 2-Pyridyl | 1 | 2.0 | 22 | 16 | 40 |
| | | 10.0 | 40 | 43 | Pt |
| 2-Pyridyl | 2 | 2.0 | 34 | 53 | 28 |
| | | 10.0 | 42 | >71 | Pt |
| C₆H₄-4-Cl | 2 | 2.0 | 24 | 20 | 20 |
| | | 10.0 | 46 | 25 | 25 |
| C₆H₄-3-Cl | 2 | 2.0 | 27 | 9 | 33 |
| | | 10.0 | 44 | 62 | 10 |
| C₆H₄-3-CF₃ | 2 | 2.0 | - | - | Pt |
| | | 10.0 | 10 | Tr | 25 |
| C₆H₄-2-C₂H₅ | 2 | 2.0 | 35 | 55 | 68 |
| | | 10.0 | 20 | 63 | R |
| C₆H₄-4-F | 2 | 2.0 | 23 | 40 | 46 |
| | | 10.0 | 33 | >84 | R |

| | | | | | |
|---|---|---|---|---|---|
| ^{a} R=Reversal; Pt=potentiation (was within 20%) | | | | | |

**Table 3: Compound administered at the time of reperfusion at a dose of 0.001 ug/ml on brief period (16 min.) of ischemic insult.**

| Compound of formula 1 | | Onset (min.) | Percentage recovery | |
|---|---|---|---|---|
| Ar | n | | 15 min. | 30 min. |
| Control | - | 2.0 | 104.50 | 118.10^{a} |
| C₆H₄-4-F | I | 1.5 | 81.25 | 78.12 |
| C₆H₄-2-C₂H₅ | 1 | 3.0 | 106.06 | 136.36 |
| C₆H₄-3-Cl | 1 | 1.0 | 50.00 | 88.80 |
| C₆H₄-4-C₂H₅ | 1 | 2.0 | 103.03 | 60.60 |
| C₆H₄-3-F | 1 | 2.0 | 120.00 | 180.00 |
| C₆H₄-3-Cl | 2 | 3.0 | 31.50 | 39.40 |
| C₆H₄-2-OCH₃ | 1 | 2.0 | 45.50 | 59.09 |
| C₆H₄-4-Cl | 1 | 1.0 | 87.50 | 120.80 |
| 2-Pyridyl | 2 | 2.0 | 47.60 | 71.40 |

| | | | | |
|---|---|---|---|---|
| a - Arrhythmia present | | | | |

The following examples are provided by the way of illustration of the present invention and should in no way be construed as a limitation thereof including the linker (propyl) between pyrrolidone/piperidone and N-arylpiperazine which may be ethyl or butyl.

### Example 1

### (a) Preparation of 1-chloro-3-[2-oxopyrrolidin-1-yl]propane

**(i)** A mixture of 2-pyrrolidone (1 g, 12.0 mmol) and finely pulverized sodium metal (0.28 g, 12.0 mmol) in dry xylene (60 ml) was heated at 110°C with vigorous stirring, 1-bromo-3-chloropropane (1.8 g, 12.0 mmol) was added to the stirred reaction mixture after 3 hours and the heating at 110°C was continued for 6 hours. The reaction mixture was filtered and xylene was removed under reduced pressure. The residue was distilled under reduced pressure to give 3 (n=1), B.P. 145°C/l mm., yield 1.52 g (80%). IR (Neat) : 2980, 2880, 1710, 1460, 1420, 1280, 1050. ¹H NMR (CDCl₃): 1.92-2.18 (m, 4H, 4' & 2-CH₂), 2.40(t, 2H, J=6.0 Hz, 3'-CH₂), 3.42(t, 4H, J=6.0 Hz, 5' & 3-CH ₂), 3.58 (t, 2H, J=6.0 Hz, 1-CH₂). MS: m/z 161 (M⁺).

| | | | | |
|---|---|---|---|---|
| Mol. formula C₇H₁₂NOCl : | Found : | C, 51.96; | H, 7.48; | N, 8.61 |
| | Calcd.: | C, 52.11; | H, 7.45; | N, 8.69%. |

**(ii)** A mixture of 2-pyrrolidone (10 g, 120.0 mmol) and finely pulverized sodium metal (2.76 g, 120.0 mmol) in dry xylene (600 ml) was heated at 150°C with vigorous stirring. 1-Bromo-3-chloropropane (18.84 g, 120.0 mmol) was added to the stirred reaction mixture after 30 minutes and the heating at 150°C was continued for 1 hour. The reaction mixture was filtered and xylene was removed under reduced pressure. The residue was distilled under reduced pressure to give 3 (n=1), B. P. 145°C/ 1 mm., yield 16.09g (85%).
**(iii)** A mixture of 2-pyrrolidone (2 g, 23.0 mmol) and finely pulverized sodium metal (0.529 g, 23.0 mmol) in dry toluene (120 ml) was heated at 120°C with vigorous stirring. 1-Bromo-3-chloropropane (3.97 g, 25.0 mmol) was added to the stirred reaction mixture after 6-7 hours and the heating at 120°C was continued for 7 hours. The reaction mixture was filtered and toluene was removed under reduced pressure. The residue was distilled under reduced pressure to give the compound 3 (n=1), B.P. 145°C/1mm., yield 2.86g (75%).
**(iv)** A mixture of 2-pyrrolidone (1 g, 12.0 mmol) and finely pulverized potassium metal (0.47 g, 12.0 mmol) in dry xylene (60 ml) was heated at 150°C with vigorous stirring. 1-Bromo-3-chloropropane (1.8 g, 12.0 mmol) was added to the stirred reaction mixture after 20 minutes and the heating at 150°C was continued for 1 hour. The reaction mixture was filtered and xylene was removed under reduced pressure. The residue was distilled under reduced pressure to give 3 (n=1), B.P. 145°/lmm., yield 1.43g (75%).
**(v)** A mixture of 2-pyrrolidone (1 g, 12.0 mmol) and potassium tert. butoxide (1.34 g, 12.0 mmol) in dry xylene (60 ml) was heated at 150°C with vigorous stirring. 1-Bromo-3-chloropropane (1.8 g, 12.0 mmol) was added to the stirred reaction mixture after 2 hours and the heating at 150°C was continued for 3 hours. The reaction mixture was filtered and xylene was removed under reduced pressure. The residue was distilled under reduced pressure to give 3 (n=1), B.P. 145°C/l mm., yield 1.24 g (65%).

### (b) Preparation of 1-chloro-3-(2-oxopiperidin-1-yl)propane

A mixture of 2-piperidone (1.19 g, 12.0 mmol) and finely pulverized sodium metal (0.28 g, 12.0 mmol) in dry xylene (70 ml) was heated at 110°C with vigorous stirring, 1-bromo-3-chloropropane (1.89 g, 12.0 mmol) was added to the stirred reaction mixture after 3 hours and the heating at 110°C was continued for 6 hours. The reaction mixture was filtered and xylene was removed under reduced pressure. The residue was chromatographed on silica gel using hexane and chloroform as eluant to get 3 (n=2), B.P. 91°C/0.01 mm., yield 1.33 g (63.33%). IR (Neat): 3862., 3298, 2950, 2474, 2324, 1640, 1478, 1432, 1336, 1184, 1096, 754. ¹H NMR (CDCl₃) 1.79-2.36 (m, 8H), 3.15-3.67 (m,6H). MS: m/z 175 (M⁺).

| | | | | |
|---|---|---|---|---|
| Mol. formula C₈H₁₄NOCL : | Found : | C, 54.90; | H, 8.28; | N, 8.25 |
| | Calcd.: | C, 54. 69; | H.8.03; | N, 7.97%. |

### Example 2

### (a) Preparation of 1-[4-(3-chlorophenyl)piperazin-1-yl]-3-[2-oxopyrrolidin-1-yl]propane of the formula 1, where Ar=C₆H₄-3-Cl, n=1

A mixture of 1-chloro-3-[2-oxopyrrolidin-1-yl]propane (1g, 6.2 mmol), 1-(3-chlorophenyl)piperazine (1.22 g, 6.2 mmol) anhydrous Na₂CO₃ (0.33 g, 3.1 mmol) and NaI (0.093 g, 0.6 mmol) in dry DMF (5 ml) was stirred at 70°C for 14 hours. The reaction mixture was cooled, poured on water (20 ml) and the separated residue was extracted with CHCl₃ (2x25 ml). The extracts were dried over Na₂SO₄ and concentrated under reduced pressure to give 1-[4-(3-chlorophenyl)piperazin-1-yl]-3-[2-oxopyrrolidin-1-yl] propane as an oil which was purified by flash column chromatography over silica gel using chloroform as eluent, yield 1.50 g (75%) . IR (Neat): 3020, 2820, 1660, 1590, 1450, 1210, 730. ¹H NMR (CDCl₃): 1.30-2.60 (m, 12H, 3', 4', 2, 1 & 2xN-CH₂), 2.40-3.50 (m, 8H, 5', 3 & 2xN-CH ₂), 6.40-7.20(m, 4H, ArH). MS: m/z 321 (M⁺) 323 (M+2).

| | | | | |
|---|---|---|---|---|
| Mol. Formula C₁₇H₂₄ClN₃O : | Found : | C, 63.58; | H, 7.82; | N, 13. 17 |
| | Calcd : | C, 63.44; | H, 7.52; | N, 13.06%. |

**(b)** A mixture of 2-pyrrolidone (1 g, 12 mmol) and finely pulverized sodium metal (0.28 g, 12.0 mmol) in dry toluene (60 ml) was heated at 120°C with vigrous stirring for 6 hours, 1-[4-(3-chlorophenyl)-piperazin-1-yl]-3-chloropropane (3.26 g, 12.0 mmol) was added to this reaction mixture and the reaction mixture was heated under stirring at 120°C for 7 hour. The reaction mixture was filtered and toluene was removed under reduced pressure. The residue was chromatographed over flash silica gel using chloroform as eluent to give the title product, yield 2.65 g (70%), oil.

**(c)** A mixture of 1-chloro-3-[2-oxopyrrolidin-1-yl]propane (1.0 g, 6.2 mmol), 1-(3-chlorophenyl)piperazine (1.22 g, 6.2 mmol), anhydrous Na₂CO₃ (0.33 g, 3.1mmol) and NaI (0.093 g, 0.6 mmol) in dry toluene (10 ml) was stirred at 110°C for 12 hrs. The solvent was removed at reduced pressure and residue was poured on water (20 ml). The separated residue was extracted with ethylacetate (3x20 ml), dried over Na₂SO₄ and concentrated under reduced pressure to give 1-[4-(3-chlorophenyl)piperazin-1-yl]-3-[2-oxopyrrolidin-1-yl]propane as an oil which was purified by flash column chromatography over silica gel using chloroform as eluent, yield 0.60 g (30.0%).

**(d)** A mixture of 1-chloro-3-[2-oxopyrrolidin-1-yl]propane (1 g, 6.2 mmol), 1-(3-chlorophenyl)piperazine (1.22 g, 6.2 mmol) anhydrous Na₂CO₃ (0.33 g, 3.1 mmol) and NaI (0.093 g, 0.6 mmol) in dry xylene (15 ml) was stirred at 150°C for 14 hrs. The solvent was removed at reduced pressure and residue was poured on water (30 ml). The separated residue was extracted with dichloromethane (2x25 ml), dried over Na₂SO₄ and concentrated to give 1-[4-(3-chlorophenyl)piperazin-1-yl]-3-[2-oxopyrrolidin-1-yl]propane as an oil which was, purified by flash column chromatography over silica gel using chloroform as eluent, yield 0.72 g (36%).

### Example 3

### (a) Preparation of 1-[4-(4-chlorophenyl)piperazin-1-yl]-3-[2-oxopyrrolidin-1-yl]propane of the formula 1, where Ar=C₆H₄-4-Cl, n=1

A mixture of 1-chloro-3-[2-oxopyrrolidin-1-yl]propane (1 g, 6.2 mmol), 1-(4-chlorophenyl)piperazine (1.22 g, 6.2 mmol) anhydrous Na₂CO₃ (0.33 g, 3.1 mmol) and NaI (0.09 g, 0.6 mmol) in dry DMF (5 ml) was stirred at 70°C for 12 hrs. The reaction mixture was cooled, poured on water (20 ml) and the separated residue was extracted with CHCl₃ (2x25 ml). The extracts were dried over Na₂SO₄ and concentrated under reduced pressure to give 1-[4-(4-chlorophenyl)piperazin-1-yl]-3-[2-oxopyrrolidin-1-yl]-propane which was purified by flash column chromatography over silica gel using chloroform as eluent, yield 1.48 g (74%), M.P. 78-80°C. IR (KBr): 3440, 2820, 1660, 1490, 1230, 1130, 810. ¹H NMR (CDCl₃): 1.50-2.80(m, 12H, 3', 4', 2, 1 & 2xN-CH₂), 3.00-3.60 (m, 8H, 5', 3 & 2xN-CH₂), 6.80 (d, 2H, J=9.0 Hz, ArH, o to Cl), 7.20 (d, 2H, J=9.0 Hz, ArH, m to Cl). MS: m/z 321 (M⁺) 323 (M+2).

| | | | | |
|---|---|---|---|---|
| Mol. formula C₁₇H₂₄ClN₃O : | Found : | C, 63.84; | H, 7.32; | N, 13.12 |
| | Calcd.: | C, 63.44; | H, 7.52; | N, 13. 06%. |

**(b)** A mixture of 2-pyrrolidone (1 g, 12 mmol) and finely pulverized sodium metal (0.28 g, 12.0 mmol) in dry xylene (60 ml) was heated at 140°C with vigrous stirring, 1-[4(4-chlorophenyl)piperazin-1-yl]-3-chloropropane (3.26 g, 12.0 mmol) was added to the stirred reaction mixture after 30 minutes and the heating at 140°C was continued for 1 hour. The reaction mixture was filtered and xylene was removed under reduced pressure to give 3 which was purified by flash column chromatography over silica gel using chloroform as eluent, yield 2.83 g (75%), M.P. 78-80°C.

**(c)** A mixture of 2-pyrrolidone (1 g, 12 mmol) and finely pulverized potassium metal (0.47 g, 12.0 mmol) in dry xylene (60 ml) was heated at 150°C with vigrous stirring, 1-[4-(4-chlorophenyl)piperazin-1-yl]-3-chloropropane (3.26 g, 12.0 mmol) was added to the stirred reaction mixture after 20 minutes and the heating at 150°C was continued for 1 hour. The reaction mixture was filtered and xylene was removed under reduced pressure to give 3 which was purified by flash column chromatography over silica gel using chloroform as eluent, yield 2.78 g (73.5%), M.P. 78-80°C.

**(d)** A mixture of 2-pyrrolidone (1 g, 12 mmol) and finely pulverized potassium tert. butoxide (1.34 g, 12.0 mmol) in dry xylene (60 ml) was heated at 150°C with vigrous stirring, 1-[4-(4-chlorophenyl)piperazin-1-yl]-3-chloropropane (3.26 g, 12.0 mmol) was added to the stirred reaction mixture after 2 hrs and the heating at 150°C was continued for 4 hour. The reaction mixture was filtered and xylene was removed under reduced pressure to give 3 which was purified by flash column chromatography over silica gel using chloroform as eluent, yield 2.46 g (65% ), M.P. 78-80°C.

### Example 4

### (a) Preparation of 1-[4-(3-fluorophenyl)piperazin-1-yl]-3-[2-oxopyrrolidin-1-yl] propane of the formula 1, where Ar=C₆H₄-3-F, n=1

A mixture of 1-chloro-3-[2-oxopyrrolidin-1-yl]propane(1 g, 6.2 mmol), 1-(3-fluorophenyl)piperazine (1.12 g, 6.2 mmol), anhydrous K₂CO₃ (0.434 g, 3.1 mmol) and KI (0.10 g, 0.6 mmol) in dry DMF (5 ml) was stirred at 90°C for 12 hrs. The reaction mixture was cooled, poured on water (25 ml) and the separated residue was extracted with CHCl₃ (2x25 ml). The extracts were dried over Na₂SO₄ and concentrated under reduced pressure to give 1-[4-(3-fluorophenyl)piperazin-1-yl]-3-[2-oxopyrrolidin-1-yl]propane as an oil which was purified by flash column chromatography over silica gel using chloroform as eluent, yield 1.32 g (69.6%). 1R (Neat): 2920, 2800, 1650, 1440, 1240, 1150, 740. ¹H NMR (CDCl₃): 1.50-2.70 (m, 12H, 3', 4', 2, 1 & 2xN-CH ₂), 3.00-3.60 (m, 8H, 5', 3 & 2xN-CH₂), 6.30-6.80 (m, 4H, ArH). MS: m/z 305 (M⁺) 307 (M+2).

| | | | | |
|---|---|---|---|---|
| Mol. formula C₁₇H₂₄FN₃O: | Found: | C, 67.22; | H, 7.72; | N, 13.97 |
| | Calcd: | C, 66.88; | H, 7.92; | N, 13.76%. |

**(b)** A mixture of 2-pyrrolidone (1g, 12 mmol) and finely pulverized sodium metal (0.28g, 12.0 mmol) in dry xylene (60 ml) was heated at 150°C with vigrous stirring, 1-[4-(3-fluorophenyl)piperazin-1-yl]-3-chloropropane (3.07g, 12 mmol) was added to the stirred reaction mixture and the heating at 150°C was continued for 1 hour. The product was obtained by the similar method as in example 3, yield 2.94g (82%), oil.

### Example 5

### (a) Preparation of 1-[4-(4-fluorophenyl)piperazin-1-yl]-3-[2-oxopyrrolidin-1-yl]propane of the formula 1, where Ar=C₆H₄-4-F, n=1

A mixture of 1-chloro-3-[2-oxopyrrolidin-1-yl]propane (4g, 25.0 mmol), 1-(4-fluorophenyl)piperazine (4.47 g, 25.0 mmol), anhydrous Na₂CO₃ (1.325 g, 12.5 mmol) and NaI (0.38g, 2.5 mmol) in dry DMF (20 ml) was stirred at 120°C for 8 hrs. The reaction mixture was cooled, poured on water (30 ml) and the separated residue was extracted with CHCl₃ (2x30 ml). The extracts were dried over Na₂SO₄ and concentrated under reduced pressure to give 1-[4-(4-fluorophenyl)piperazin-1-yl]-3-[2-oxopyrrolidin-1-yl]-propane as an oil which was purified by flash column chromatography over silica gel using chloroform as eluent, yield 5.72 g (75.5%). IR (Neat): 2920, 2820, 1660, 1500, 1250, 730. ¹H NMR (CDCl₃): 1.50-2.80 (m, 12H, 3', 4', 2, 1 & 2xN-CH ₂), 3.00-3.60(m, 8H, 5', 3 & 2xN-CH₂), 6.70-7.10(m, 4H, ArH). MS: m/z 305 (M⁺).

| | | | | |
|---|---|---|---|---|
| Mol. formula C₁₇H₂₄FN₃O : | Found : | C, 66.55; | H, 8.07; | N, 13.69 |
| | Calcd.: | C, 66.86; | H, 7.92; | N, 13.76% |

**(b)** A mixture of 2-pyrrolidone (3g, 35 mmol) and finely pulverized sodium metal (0.81 g, 35.0 mmol) in dry xylene (180 ml) was heated at 150°C with vigrous stirring, 1-[4-(4-fluorophenyl)piperazin-1-yl]-3-chloropropane (8.96 g, 35.0 mmol) was added to the stirred reaction mixture after 30 minutes and the heating at 150°C was continued for 1 hour. The reaction mixture was filtered and xylene was removed under reduced pressure to give 3 which was purified by flash column chromatography over silica gel using chloroform as eluent, yield 9.15 g (85%), oil.

### Example 6

### (a) Preparation of 1-[4-(4-ethylphenyl)piperazin-1-yl]-3-[2-oxopyrrolidin-1-yl]propane of the formula 1, where Ar=C₆H₄-4-C₂H₅, n=1

A mixture of 1-chloro-3-[2-oxopyrrolidin-1-yl]propane (2.0 g, 12.0 mmol), 1-(4-ethylphenyl)piperazine (2.36 g, 12.0 mmol), anhydrous Na₂CO₃ (0.658 g, 6.2 mmol) and NaI (0.18 g, 1.2 mmol) in dry DMF (10 ml) was stirred at 80°C for 12 hrs. The reaction mixture was cooled, poured on water (30 ml) and the separated residue was extracted with CHCl₃ (2X30ml). The extracts were dried over Na₂SO₄ and concentrated under reduced pressure to give 1-[4-(4-ethylphenyl)piperazin-1-yl]-3-[2-oxopyrrolidin-1-yl]propane as an oil which was purified by flash column chromatography over silica gel using chloroform as eluent, yield 2.43 g (62.0%). 1R (Neat): 2940, 2800, 1660, 1440, 1000, 900, 800. ¹H NMR (CDCl₃): 1.20(t, 3H, J=6.0 Hz, CH₂CH₃ 1.60-2.80(m, 14H, 3',4',2,1 & 2xN-CH₂, CH₂CH₃), 3.00-3.60 (m, 8H, 5', 3 & 2xN-CH ₂). MS: m/z 315 (M⁺).

| | | | | |
|---|---|---|---|---|
| Mol. formula C₁₉H₂₉N₃O: | Found : | C, 71.94; | H, 9.16; N, | 13.15 |
| | Calcd.: | C, 72.34; | H, 9.27; | N, 13.32%. |

**(b)** A mixture of 2-pyrrolidone (2 g, 24 mmol) and finely pulverized sodium metal (0.56 g, 24.0 mmol) in dry xylene (120 ml) was heated at 150°C with vigrous stirring, 1-[4-(4-ethylphenyl)piperazin-1-yl]-3-chloropropane (6.38g, 24.0mmol) was added to the stirred reaction mixture after 30 minutes and the heating at 150°C was continued for 1 hour. The product was obtained by the similar method as in example-3, yield 6.42g (78%), oil.

### Example 7

### (a) Preparation of 1-[4-(2-ethylphenyl)piperazin-1-yl]-3-[2-oxopyrrolidin-1-yl]propane of the formula 1, where Ar=C₆H₄₋2-C₂H₅, n=1

A mixture of 1-chloro-3-[2-oxopyrrolidin-1-yl]propane (2.0 g, 12.0 mmol), 1-(2-ethylphenyl)piperazine (2.36g, 12.0 mmol), anhydrous Na₂CO₃ (0.658 g, 6.2 mmol) and NaI (0.18 g, 1.2 mmol) in dry DMF (10 ml) was stirred at 80°C for 12 hrs. The reaction mixture was cooled, poured on water (30 ml) and the separated residue was extracted with CHCl₃ (2X20ml). The extracts were dried over NaS₂O₄ and concentrated under reduced pressure to give 1-[4-(4-ethylphenyl)piperazin-1-yl]-3-[2-oxopyrrolidin-1-yl]propane as an oil which was purified by flash column chromatography over silica gel using chloroform as eluent, yield 2.17 g (60.0%). IR (Neat): 2940, 2820, 1660, 1430, 1010, 900, 800. ¹H NMR (CDCl₃): 1.24(t, 3H, J=6 Hz, CH₂-CH₃), 1.68-1.85(m, 2H, 4'-CH₂), 1.98-2.10 (m, 2H, 2-CH₂), 2.34-2.50 (m, 4H, 3' & 1-CH₂), 2.62(bs, 4H, 2xN-CH₂), 2.68(q, 2H, CH₃-CH₂), 2.94(t, 4H, J=6.0 Hz, 2xN-CH ₂), 2.35(t, 2H, J=6.0 Hz, 3-CH₂). 3.42(t 2H, J=6.0 Hz, 5'-CH₂), 7.00-7.28(m, 4H, Ar-H). MS: m/z 315 (M⁺).

| | | | | |
|---|---|---|---|---|
| Mol. formula C₁₉H₂₉N3O : | Found : | C, 72.64; | H, 9.11; | N, 13.64 |
| | Calcd.: | C, 72.34; | H, 9.27; | N, 13.32% |

**(b)** A mixture of 2-pyrrolidone (1g, 12 mmol) and finely pulverized sodium metal (0.28g, 12.0 mmol) in dry xylene (60 ml) was heated at 150°C with vigrous stirring, 1-[4-(3-ethylphenyl)piperazin-1-yl]-3-chloropropane (3.19 g, 12.0 mmol) was added to the stirred reaction mixture after 30 minutes and the heating at 150°C was continued for 1 hour. The product was obtained by the similar method as in example-3, yield 3. 13 g (76%), oil.

### Example 8

### (a) Preparation of 1-[4-(4-chlorophenyl)piperazin-1-yl]-3-[2-oxopiperidin-1-yl]propane of the formula 1, where Ar=C₆H₄-4-Cl, n=2

A mixture of 2-piperidone (1g, 10 mmol) and finely pulverized sodium metal (0.23 g, 10 mmol) in dry xylene (60 ml) was heated at 140°C with vigrous stirring, 1-[4-(4-chlorophenyl)piperazin-1-yl]-3-chloropropane (2.72 g, 10 mmol) was added to the stirred reaction mixture after 30 minutes and the heating at 140°C was continued for 1 hour. The reaction mixture was filtered and xylene was removed under reduced pressure to give 3 which was purified by flash column chromatography over silica gel using chloroform as eluent, yield 2.54 g (75%), m.p. 106°C. IR (Neat): 3761, 3408, 3017, 2957, 2882, 2826, 2785, 2502, 1659, 1599, 1499, 1456, 1385, 1346, 1219, 1148, 1105, 760, 667. PMR (CDCl₃): 0.89-1.80(m, 6H, 4',5' & 2-CH₂), 2.33-2.65(m, 8H, 3',1 & 2xN-CH₂), 3.15-3.62(m, 8H, 6',3 & 2xN-CH₂), 6.81-7.00(2xdd, 4xArH). MS: m/z 337 (M+2).

| | | | | |
|---|---|---|---|---|
| Mol. formula C₁₈H₂₆N₃OCl : | Found: | C, 64.23; | H, 7.87; | N, 12.24 |
| | Calcd .: | C, 64.37; | H, 7.80; | N, 12.51%. |

**(b)** A mixture of 2-piperidone (1.0 g, 10 mmol) and finely pulverized potassium tert. butoxide (1.12 g, 10 mmol) in dry xylene (60 ml) was heated at 150°C with vigrous stirring, 1-[4-(4-chlorophenyl)piperazin-1-yl]-3-chloropropane (2.72 g, 10 mmol) was added to the stirred reaction mixture after 2 hours and the heating at 150°C was continued for 4-hour. The reaction mixture was filtered and xylene was removed under reduced pressure to give 3 which was purified by flash column chromatography over silica gel using chloroform as eluent, yield 2.17g (64%), m.p. 106°C.

### Example 9

### Preparation of 1-[4-(3-chlorophenyl)piperazin-1-yl]-3-[2-oxopiperidin -1-yl]propane of the formula 1, where Ar=C₆H₄-3-Cl, n=2

A mixture of 2-piperidone (1.0 g, 10 mmol) and finely pulverized sodium metal (0.23 g, 10 mmol) in dry toluene (60 ml) was heated at 120°C with vigrous stirring for 6 hours, 1-[4-(3-chlorophenyl)piperazin-1-yl]-3-chloropropane (2.72g, 10 mmol) was added to this reaction mixture and the reaction mixture was heated under stirring at 120°C for 7 hour. The reaction mixture was filtered and toluene was removed under reduced pressure. The residue was chromatographed over flash silica gel using chloroform as eluent to give 3, yield 2.59 g (76.5%), oil. IR (Neat): 3406, 2951, 2878, 1626, 1597, 1491, 1454, 1383, 1352, 1219, 1142, 1103. ¹H NMR (CDCl₃): 1.17-1.84 (m, 6H, 2, 4' & 5'-CH ₂), 2.27-2.65(m, 8H, 1, 3' & 2xN-CH₂), 3.15-3.37(m, 8H, 6', 3 & 2xN-CH₂), 6.68-6.79 (dd, 2xArH), 7.05-7.1 (t, 1xArH), 7.19 (s, 1xArH). MS: m/z 335 (M⁺).

| | | | | |
|---|---|---|---|---|
| Mol. formula C₁₈H₂₆N₃OCl : | Found: | C, 64.17; | H, 7.92; | N, 12.21 |
| | Calcd.: | C, 64.37; | H, 7.80; | N, 12.51% |

### Example 10

### (a) Preparation of 1-[4-(4-fluorophenyl)piperazin-1-yl]-3-[2-oxopiperidin-1-yl]propane of the formula 1, where Ar=C₆H₄-4-F, n=2

A mixture of 2-piperidone (2g, 20 mmol) and finely pulverized sodium metal (0.46 g, 20 mmol) in dry xylene (120 ml) was heated at 150°C with vigrous stirring, 1-[4-(4-fluorophenyl)piperazin-1-yl]-3-chloropropane (5.17 g, 20 mmol) was added to the stirred reaction mixture after 30 minutes out the heating at 150°C was continued for 1 hour. The reaction mixture was filtered and xylene was removed under reduced pressure to give 3 which was purified by flash column chromatography over silica gel using chloroform as eluent, yield 4.64 g (72%), oil. IR (Neat): 3408, 3014, 2931, 2880, 2825, 1626, 1508, 1458, 1219, 824, 760, 667. ¹H NMR (CDCl₃): 1.72-1.86(m, 6H, 4', 5' & 2-CH₂)2.37-2.69 (m, 8H, 3', 1 & 2xN-CH₂), 3.12-3.46(m, 8H, 6', 3 & 2xN-CH₂), 6.83-7.00 (2xdd, 4xArH). MS: 319 (M⁺).

| | | | | |
|---|---|---|---|---|
| Mol. formula C₁₉H₂₆N₃OF : | Found : | C, 71.23; | H, 8.26; | N, 12.95 |
| | Calcd.: | C, 71.47; | H, 8.15; | N, 13.17%. |

**(b)** a mixture of 1-chloro-3-[2-oxopiperidin-1-yl]propane (2.1g, 12 mmol), 1-(4-fluorophenyl)piperazine (2.16g, 12 mmol), anhydrous Na₂CO₃ (0.618g, 6.2 mmol) and Na (0.18g, 1.2 mmol) in dry DMF (10 ml) was stirred at 80°C for 14 hrs. The reaction mixture was cooled, poured on water (30 ml) and the separated residue was extracted with CHCl₃ (2x35 ml). The extracts were dried over Na₂SO₄ and concentrated under reduced pressure to give 1-[4-(4-fluorophenyl)piperazin-1-yl]-3-[2-oxopiperidin-1-yl] propane as an oil which was purified by column chromatography over silica gel using chloroform as eluent, yield 2.40 g (63.0%)

### Example 11

### Preparation of 1-[4-(Z-ethylphenyll)piperazin-1-yl]-3-[2-oxopiperidin -1-yl]propane of the formula 1, where Ar=C₆H₄-2-C₂H₅, n=2

A mixture of 2-piperidone (1.0 g, 10 mmol) and finely pulverized sodium metal (0.23g, 10 mmol) in dry xylene (60ml) was heated at 150°C with vigrous stirring, 1-[4-(2-ethylphenyl)piperazin-1-yl]-3-chloropropane (2.66 g, 10 mmol) was added to the stirred reaction mixture after 30 minutes and the heating at 150°C was continued for 1 hour. The reaction mixture was filtered and xylene was removed under reduced pressure to give 3 which was purified by flash column chromatography on silica gel using chloroform as eluent, yield 2.49 g (75%), oil. IR (Neat): 3680, 3440, 3000, 2960, 2870, 2820, 1620, 1490, 1450, 1350, 1210, 1140, 1005, 920, 725. ¹H NMR (CDCl₃): 1.00 (t, 3H, CH₂CH₃), 1.20-1.90 (m, 6H, 4', 5' & 2-CH₂), 2.20-3.50(m, 18H, 3', 6', 3, 1 & 4xN-CH₂, CH₂CH₃), 7.00-7.20(m, 4H, ArH). MS: m/z 329 (M⁺).

| | | | | |
|---|---|---|---|---|
| Mol. formula C₂₀H₃₁N₃O : | Found : | C, 72.67; | H, 9.60; | N, 12.49 |
| | Calcd.: | C, 72.95; | H, 9.42; | N, 12.77%. |

### Example 12

### Preparation of 1-[4-(2-methoxyphenyl)piperazin-1-yl]-3-[2-oxopiperidin-1-yl]propane of the formula 1, where Ar=C₆H₅-2-OCH₃, n=2

A mixture of 2-piperidone (1.0 g, 10 mmol) and finely pulverized potassium metal (0.40 g, 10 mmol) in dry xylene (60 ml) was heated at 150°C with vigrous stirring, 1-[4-(2-methoxyphenyl)piperazin-1-yl]-3-chloropropane (2.52 g, 10 mmol) was added to the stirred reaction mixture after 20 minutes and the heating at 150°C was continued for 1 hour. The reaction mixture was filtered and xylene was removed under reduced pressure to give 3 which was purified by flash column chromatography over silica gel using chloroform as eluent, yield 2.41g (72%), oil. IR (Neat): 2945, 1626, 1500, 1460, 1242, 908, 735, 648. PMR (CDCl₃): 1.69-1.84(m, 6H, 4', 5' & 2-CH₂), 2.26-2.70(m, 8H, 3', 1 & 2xN-CH₂), 3.07-3.36 (m, 8H, 6', 3 & 2xN-CH₂), 3.75 (s, 3H, OCH₃), 6.76-6.95 (m, 4H, ArH). MS: m/z 331 (M⁺).

| | | | | |
|---|---|---|---|---|
| Mol. formula C₁₉H₂₉N₃O₂ : | Found : | C, 68.61; | H, 8.92; | N, 12.56 |
| | Calcd .: | C, 68.85; | H, 8.81; | N, 12 68%. |

### Example 13

### Preparation of 1-[4-(2-pyridyl)piperazin-1-yl]-3-(2-oxopiperidin-1-yl] propane of the formula 1, where Ar=2-pyridyl, n=2

A mixture of 2-piperidone (2g, 20 mmol) and finely pulverized sodium metal (0.46 g, 20 mmol) in dry xylene (120 ml) was heated at 150°C with vigrous stirring, 1-[4-(2-pyridyl)piperazin-1-yl]-3-chloropropane (4.78g, 20 mmol) was added to the reaction mixture after 30 minutes and the heating at 150°C was continued for 1 hour. The reaction mixture was filtered and xylene was removed under reduced pressure to give 3 which was purified by flash column chromatography over silica gel using chloroform as eluent, yield 4.39g (72%), oil. IR (Neat): 3420, 2940, 2800, 1629, 1580, 1470, 1420, 1230, 1150, 1125, 960, 720. ¹H NMR (CDCl₃): 1.50-2.20 (m, 6H, 4', 5' & 2-CH₂), 2.25-2.80(m, 8H, 3', 1 & 2xN-CH₂), 3.10-4.80 (m, 8H, 6', 3 & 2xN-CH₂), 6.48-7.67 (m, 2H, 3,5-pyridyl H), 7.40 (m, 1H, 4-pyridyl H), 8.15 (m, 1H, 6-pyridyl H). MS: m/z 302 (M⁺).

| | | | | |
|---|---|---|---|---|
| Mol. formula C₁₇H₂₆N₄ : | Found : | C, 67.32; | H, 8.49; | N, 18.38 |
| | Calcd .: | C, 67.52; | H, 8.67; | N, 18.53%. |

### Example 14

### Preparation of 1-[4-(3-trifluoromethylphenyl)piperazin-1-yl]-3-[2-oxopiperidin-1-yl]propane of the formula 1, where Ar=C₆H₄-3-CF₃, n=2

A mixture of 2-piperidone (1.0 g, 10 mmol) and finely pulverized sodium metal (0.23 g, 10 mmol) in dry xylene (60 ml) was heated at 150°C with vigorous stirring, 1-[4-(3-CF₃-phenyl) piperazin-1-yl]-3-chloropropane (3.06 g, 10 mmol) was added to the stirred reaction mixture after 30 minutes and the heating at 150°C was continued for I hour. The reaction mixture was filtered and xylene was furnished by flush column chromatography on silica gel using CHCl₃ as eluent, yield 2.80 g (75%), oil. IR (Neat): 3402, 3015, 2951, 2880, 2827, 2785, 1622, 1449, 1450, 1352 1315, 1217, 1167, 1126, 1076, 997, 951. ¹H NMR (CDCl₃): 1.75-1.93 (m, 6H, 4', 5' & 2-CH₂), 2.35 -2.75 (m, 8H, 3', 1 & 2xN-CH₂), 3.310-3.84 (m, 8H, 6', 3 & 2xN-CH₂), 7.04-7.10 (dd, 2H, 4, 6-ArH), 7.26 (s, H, 2-ArH), 7.32-7.37 (t, H, 5-ArH). MS: m/z 369 (M⁺)

| | | | | |
|---|---|---|---|---|
| Mol. formula C₁₉H₂₆N₃OF₃⁻: | Found: | C, 61.48; | H, 7.23; | N, 11.21 |
| | Calcd.: | C, 61.79; | H, 7.05; | N, 11.38% |

### Example 15

### Antihypertensive/hypotensive activity

**(a)** Cats (2.6-4.0 kg) of either sex anaesthetized with pentobarbitone sodium (40 mg/kg i.v.) and showing basal mean arterial blood pressure below 150 mm (Hg) were categorised as normotensive and above 150 mm Hg as hypertensive. Arterial blood pressure (BP) was recorded from one of the carotid artery through a stathum P23 DC pressure transducer and 7P1 low level DC preamplifier on a Grass Model P7 Polygraph, Signals from 7P 1 preamplifier were used to trigger 7P4 Tachograph preamplifier for recording the heart rate (HR). Right femoral vein and Trachea were cannulated for intravenous injections and artificial ventilation respectively. Control responses to intravenous injection of noradrenaline (2-4 ug); acetyl choline (1-2 ug); histamine (1-2 ug) and isoprenaline (1-2 ug) were taken before and after the administration of test doses of each compounds. All the compounds were tested at fixed doses of 2.0 and 10 uM/kg i.v. Significant results are given in Table 2.
**(b)** Antihypertensive activity was observed in naturally hypertensive cats, rat abdominal aorta coarctation model of hypertension (Liu, J; Bishop, S.P. & Overbeck, H.W. Morphometric evidence for non pressure related arterial wall thickening in hypertension Circ. Res. 62, 1001-1010, 1988) and L-NAME (No synthase inhibitor) induced hypertensive cats.

### Example 16

### Cardioprotective/antiischemic activity

The rats were killed by decapitation, heart were rapidly excised and placed in ice-cold HEPES tyrode buffer. Then isolated hearts were perfused retrogradely through coronary arteries using the Langendorff technique. The perfusion buffer consisted (in mM) NaCl 137, KCl 5.4, CaCl₂ 1.8, MgCl ₂ 1.0, glucose 11.2 and HEPES 3.0. The buffer (pH 7.4) was continuously gassed with oxygen and maintained at 37°C. Coronary flow rate was maintained at 10 ml/min. The heart contracted spontaneously.
The perfused heart was allowed to equilibrate for 30 min. before initiation of any insult protocol. The test compounds were given at the time of reperfusion. Some of the compounds were dissolved in ethanol. Final concentration of ethanol in the perfusion buffer was 0.0001% and had no effect of its own on the parameter used.
**(a)** For brief period of ischemic insult (Hideo *et al.* Pathophysiology and pathogenesis of stunned myocardium. *J. Clin. Invest*., 79, 950-961, 1987). Ischemia was initiated by stopping the flow for 16 min. followed by 30 min. reperfusion period. The durations were decided on the initial pilot experiments leading to 50% recovery of function.
**(b)** For prolong period of ischemic insult (Becker, L.C. & Ambrosio, G. Myocardial consequences of reperfusion *Prog. Cardiovas. Dis.,* 30, 23-44, 1987). Ischemia was initiated by stopping the flow for 30 min. followed by 30 min. reperfusion.

## Claims

1. A process for the synthesis of compounds of formula I 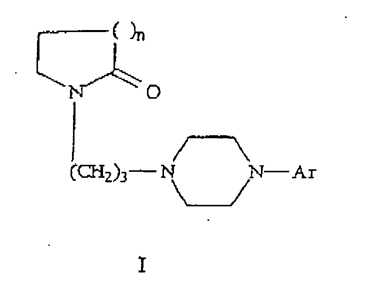 wherein Ar represents pyridyl or phenyl, or a phenyl group substituted by one or more alkyl, alkoxy or halogen groups, or by one or more CF₃ groups, n=1 or n=2; which process comprises condensing 2-pyrrolidone of formula 2 (n=1) or 2-piperidone of formula 2 (n=2) with 1-[4-substituted arylpiperazin-1-yl]-3-chloropropanes of formula 5 where Ar is above in the presence of a base and organic solvent at a temperature ranging from 120-150°C for a period varying between 90 min to 14 hrs. to produce the corresponding 1-[4-substituted arylpiperazin-1-yl]-3-[2-oxopyrrolidin/piperidin-1-yl]propanes of formula I.

2. A process as claimed in claim 1 wherein the preferred compounds are defined herebelow:
(a) 1-[4-(3-chlorophenyl)piperazin-1-yl]-3-[2-oxopyrrolidin-1-yl] propane.
(b) 1-[4-(4-chlorophenyl)piperazin-1-yl]-3-[2-oxopyrrolidin-1-yl] propane.
(c) 1-[4-(3-fluorophenyl)piperazin-1-yl]-3-[2-oxopyrrolidin-1-yl] propane.
(d) 1-[4-(4-fluorophenyl)piperazin-1-yl]-3-[2-oxopyrrolidin-1-yl] propane.
(e) 1-[4-(4-ethylphenyl)piperazin-1-yl]-3-[2-oxopyrrolidin-1-yl] propane.
(f) 1-[4-(2-ethylphenyl)piperazin-1-yl]-3-[2-oxopyrrolidin-1-yl] propane.
(g) 1-[4-(4-chlorophenyl)piperazin-1-yl]-3-[2-oxopiperidin-1-yl] propane.
(h) 1-[4-(3-chlorophenyl)piperazin-1-yl]-3-[2-oxopiperidin-1-yl] propane.
(i) 1-[4-(4-fluorophenyl)piperazin-1-yl]-3-[2-oxopiperidin-1-yl] propane.
(j) 1-[4-(2-ethylphenyll)piperazin-1-yl]-3-[2-oxopiperidin-1-yl] propane.
(k) 1-[4-(2-methoxyphenyl)piperazin-1-yl]-3-[2-oxopiperidin-1-yl] propane.
(l) 1-[4-(2-pyridyl)piperazin-1-yl]-3-[2-oxopiperidin-1-yl]propane.
(m) 1-[4-(3-trifluoromethylphenyl)piperazin-1-yl]-3-[2-oxopiperidin-1-yl] propane.

3. A process as claimed in claim 1 wherein the molar ratio of the compounds of formula 2 and 5 is 1:1.

4. A process as claimed in claim 1 wherein the solvent used is selected from toluene or xylene and the amount of the solvent used ranges from 5 to 6ml per mmol of the reacting compounds of formula 2 and 5.

5. A process as claimed in claim 1 where atomic/molar ratio of the base sodium/potassium metal or potassium *tert.* butoxide to the compounds of formula 2 and 5 is 1:1.

6. A pharmaceutical composition comprising a compound of formula 1 as defined in claim 1 in admixture with a pharmaceutically acceptable conventional carrier.

7. A process for the preparation of a pharmaceutical composition which comprises bringing a compound of the formula 1 as defined in claim 1 into association with a pharmaceutically acceptable carrier.

8. The use of a compound of formula 1, as defined in claim 1, in the manufacture of a medicament for treating any of the following disorders: hypertension in mammals, peripheral vascular diseases in mammals, diseases arising out of alterations/impairment in central/peripheral circulations and adrenergic receptors systems, such as myocardial ischemia, myocardial infarction (MI), angina pectoris, any cardiac surgical interventions, renal ischemia, circulatory insufficiency in extremities, stroke and trauma, reperfusion injury in mammals, ischemic diseases like myocardial infarction (MI) or angina pectoris.

9. A compound of formula I 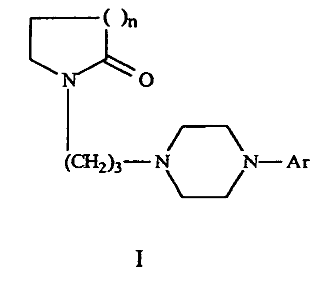 wherein Ar represents pyridyl or phenyl, or a phenyl group substituted by one or more alkyl, alkoxy or halogen groups, or by one or more CF₃ groups, n=1 or n=2, including the compounds:
(a) 1-[4-(3-chlorophenyl)piperazin-1-yl]-3-[2-oxopyrrolidin-1-yl] propane.
(b) 1-[4-(4-chlorophenyl)piperazin-1-yl]-3-[2-oxopyrrolidin-I-yl] propane.
(c) 1-[4-(3-fluorophenyl)piperazin-1-yl]-3-[2-oxopyrrolidin-1-yl] propane.
(d) 1-[4-(4-fluorophenyl)piperazin-1-yl]-3-[2-oxopyrrolidin-1-yl] propane.
(e) 1-[4-(4-ethylphenyl)piperazin-1-yl]-3-[2-oxopyrrolidin-1-yl] propane.
(f) 1-[4-(2-ethylphenyl)piperazin-1-yl]-3-[2-oxopyrrolidin-1-yl] propane.
(g) 1-[4-(4-chlorophenyl)piperazin-1-yl]-3-[2-oxopiperidin-1-yl] propane.
(h) 1-[4-(3-chlorophenyl)piperazin-1-yl]-3-[2-oxopiperidin-1-yl] propane.
(i) 1-[4-(4-fluorophenyl)piperazin-1-yl]-3-[2-oxopiperidin-1-yl] propane.
(j) 1-[4-(2-ethylphenyll)piperazin-1-yl]-3-[2-oxopiperidin-1-yl] propane.
(k) 1-[4-(2-methoxyphenyl)piperazin-1-yl]-3-[2-oxopiperidin-1-yl] propane.
(l) 1-[4-(2-pyridyl)piperazin-1-yl]-3-[2-oxopiperidin-1-yl]propane.
(m) 1-[4-(3-trifluoromethylphenyl)piperazin-1-yl]-3-[2-oxopiperidin-1-yl] propane.

10. A compound according to claim 9 for use as a medicament.

## Patentansprüche

1. Verfahren zur Synthese von Verbindungen mit der Formel I 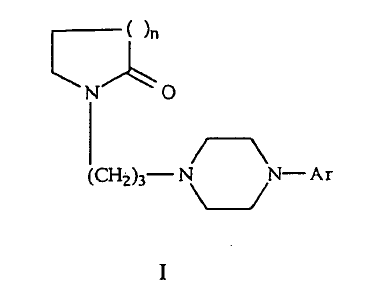 wobei Ar Pyridyl oder Phenyl oder eine Phenylgruppe, die mit einer oder mehreren Alkyl-, Alkoxy- oder Halogengruppen oder mit einer oder mehreren CF₃-Gruppen substituiert ist, darstellt, n=1 oder n=2; wobei dieses Verfahren das Kondensieren von 2-Pyrrolidon mit der Formel 2 (n=1) oder 2-Piperidon mit der Formel 2 (n=2) mit 1-[4-substituierten Arylpiperazin-1-yl]-3-chlorpropanen mit der Formel 5 umfasst, wobei Ar wie oben ist, in Anwesenheit einer Base und eines organischen Lösungsmittels bei einer Temperatur, die im Bereich von 120-150°C liegt, über einen Zeitraum, der zwischen 90 Minuten bis 14 Stunden schwankt, um die entsprechenden 1-[4-substituierten Arylpiperazin-1-yl]-3-[2-oxopyrrolidin/piperidin-1-yl]-propane mit der Formel I zu erzeugen.

2. Verfahren wie in Anspruch 1 beansprucht, wobei die bevorzugten Verbindungen hier untenstehend definiert sind:
(a) 1-[4-(3-Chlorphenyl)piperazin-1-yl]-3-[2-oxopyrrolidin-1-yl]-propan,
(b) 1-[4-(4-Chlorphenyl)piperazin-1-yl]-3-[2-oxopyrrolidin-1-yl]-propan,
(c) 1-[4-(3-Fluorphenyl)piperazin-1-yl]-3-[2-oxopyrrolidin-1-yl]-propan,
(d) 1-[4-(4-Fluorphenyl)piperazin-1-yl]-3-[2-oxopyrrolidin-1-yl]-propan,
(e) 1-[4-(4-Ethylphenyl)piperazin-1-yl]-3-[2-oxopyrrolidin-1-yl]-propan,
(f) 1-[4-(2-Ethylphenyl)piperazin-1-yl]-3-[2-oxopyrrolidin-1-yl]-propan,
(g) 1-[4-(4-Chlorphenyl)piperazin-1-yl]-3-[2-oxopiperidin-1-yl]-propan,
(h) 1-[4-(3-Chlorphenyl)piperazin-1-yl]-3-[2-oxopiperidin-1-yl]-propan,
(i) 1-[4-(4-Fluorphenyl)piperazin-1-yl]-3-[2-oxopiperidin-1-yl]-propan,
(j) 1-[4-(2-Ethylphenyl)piperazin-1-yl]-3-[2-oxopiperidin-1-yl]-propan,
(k) 1-[4-(2-Methoxyphenyl)piperazin-1-yl]-3-[2-oxopiperidin-1-yl]-propan,
(l) 1-[4-(2-Pyridyl)piperazin-1-yl]-3-[2-oxopiperidin-1-yl]-propan,
(m) 1-[4-(3-Trifluormethylphenyl)piperazin-1-yl]-3-[2-oxopiperidin-1-yl]-propan.

3. Verfahren wie in Anspruch 1 beansprucht, wobei das Molverhältnis der Verbindungen mit den Formeln 2 und 5 1:1 beträgt.

4. Verfahren wie in Anspruch 1 beansprucht, wobei das verwendete Lösungsmittel aus Toluol oder Xylol ausgewählt ist und die Menge des verwendeten Lösungsmittels im Bereich von 5 bis 6 ml pro mmol der reagierenden Verbindungen mit den Formeln 2 und 5 liegt.

5. Verfahren wie in Anspruch 1 beansprucht, wobei das Atom-/Molverhältnis der Base Natrium/Kaliummetall oder Kalium-*tert*-butoxid zu den Verbindungen mit den Formeln 2 und 5 1:1 beträgt.

6. Pharmazeutische Zusammensetzung, die eine Verbindung mit der Formel 1 wie in Anspruch 1 definiert in Beimischung mit einem pharmazeutisch akzeptablen herkömmlichen Träger umfasst.

7. Verfahren zum Herstellen einer pharmazeutischen Zusammensetzung, das es umfasst, eine Verbindung mit der Formel 1 wie in Anspruch 1 definiert mit einem pharmazeutisch akzeptablen Träger in Verbindung zu bringen.

8. Verwendung einer Verbindung mit der Formel 1 wie in Anspruch 1 definiert bei der Herstellung eines Medikaments zum Behandeln einer der folgenden Erkrankungen: Hypertension bei Säugern, periphere Gefäßerkrankungen bei Säugern, Krankheiten, die sich aus Veränderungen/Beeinträchtigung von zentralen/peripheren Kreisläufen und adrenergen Rezeptorsystemen ergeben, wie zum Beispiel Myokardischämie, Myokardinfarkt (MI), Angina pectoris, alle herzchirurgischen Interventionen, renale Ischämie, unzureichende Durchblutung in Extremitäten, Schlaganfall und Verletzung, Reperfusionsschaden bei Säugern, ischämische Erkrankungen wie Myokardinfarkt (MI) oder Angina pectoris.

9. Verbindung mit der Formel I 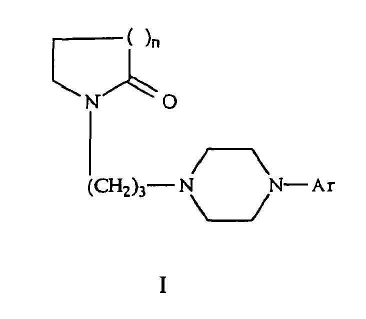 wobei Ar Pyridyl oder Phenyl oder eine Phenylgruppe, die mit einer oder mehreren Alkyl-, Alkoxy- oder Halogengruppen oder mit einer oder mehreren CF₃-Gruppen substituiert ist, darstellt, n=1 oder n=2, einschließlich der Verbindungen:
(a) 1-[4-(3-Chlorphenyl)piperazin-1-yl]-3-[2-oxopyrrolidin-1-yl]-propan,
(b) 1-[4-(4-Chlorphenyl)piperazin-1-yl]-3-[2-oxopyrrolidin-1-yl]-propan,
(c) 1-[4-(3-Fluorphenyl)piperazin-1-yl]-3-[2-oxopyrrolidin-1-yl]-propan,
(d) 1-[4-(4-Fluorphenyl)piperazin-1-yl]-3-[2-oxopyrrolidin-1-yl]-propan,
(e) 1-[4-(4-Ethylphenyl)piperazin-1-yl]-3-[2-oxopyrrolidin-1-yl]-propan,
(f) 1-[4-(2-Ethylphenyl)piperazin-1-yl]-3-[2-oxopyrrolidin-1-yl]-propan,
(g) 1-[4-(4-Chlorphenyl)piperazin-1-yl]-3-[2-oxopiperidin-1-yl]-propan,
(h) 1-[4-(3-Chlorphenyl)piperazin-1-yl]-3-[2-oxopiperidin-1-yl]-propan,
(i) 1-[4-(4-Fluorphenyl)piperazin-1-yl]-3-[2-oxopiperidin-1-yl]-propan,
(j) 1-[4-(2-Ethylphenyl)piperazin-1-yl]-3-[2-oxopiperidin-1-yl]-propan,
(k) 1-[4-(2-Methoxyphenyl)piperazin-1-yl]-3-[2-oxopiperidin-1-yl]-propan,
(l) 1-[4-(2-Pyridyl)piperazin-1-yl]-3-[2-oxopiperidin-1-yl]-propan,
(m) 1-[4-(3-Trifluormethylphenyl)piperazin-1-yl]-3-[2-oxopiperidin-1-yl]-propan.

10. Verbindung nach Anspruch 9 zur Verwendung als Medikament.

## Revendications

1. Procédé pour la synthèse de composés de formule I 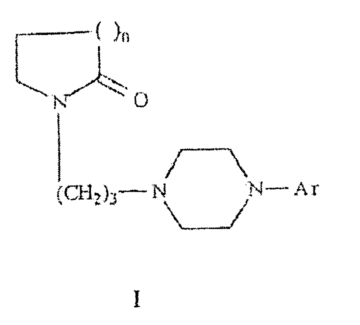 dans laquelle Ar représente pyridyle ou phényle, ou un groupe phényle substitué par un ou plusieurs groupes alkyle, alcoxy ou halogè ne, ou par un ou plusieurs groupes CF₃, n = 1 ou n = 2 ; lequel procédé comprend la condensation de 2-pyrrolidone de formule 2 (n = 1) ou de 2-pipéridone de formule 2 (n = 2) avec des 1-[4-arylpipérazin-1-yl-substitué]-3-chloropropanes de formule 5 où Ar est ci-dessus en présence d'une base et d'un solvant organique à une température allant de 120 à 150°C pendant une période variant entre 90 min à 14 heures pour produire les 1-[4-arylpipérazin-1-yl-substitué]-3-[2-oxypyrrolidin/pipéridin-1-yl]propanes de formule I correspondants.

2. Procédé selon la revendication 1, dans lequel les composés préférés sont définis ci-dessous :
(a) 1-[4-(3-chlorophényl)pipérazin-1-yl]-3-[2-oxypyrrolidin-1-yl]propane,
(b) 1-[4-(4-chlorophényl)pipérazin-1-yl]-3-[2-oxypyrrolidin-1-yl]propane,
(c) 1-[4-(3-fluorophényl)pipérazin-1-yl]-3-[2-oxypyrrolidin-1-yl]propane,
(d) 1-[4-(4-fluorophényl)pipérazin-1-yl]-3-[2-oxypyrrolidin-1-yl]propane,
(e) 1-[4-(4-éthylphényl)pipérazin-1-yl]-3-[2-oxypyrrolidin-1-yl]propane,
(f) 1-[4-(2-éthylphényl)pipérazin-1-yl]-3-[2-oxypyrrolidin-1-yl]propane,
(g) 1-[4-(4-chlorophényl)pipérazin-1-yl]-3-[2-oxypipéridin-1-yl]propane,
(h) 1-[4-(3-chlorophényl)pipérazin-1-yl]-3-[2-oxypipéridin-1-yl]propane,
(i) 1-[4-(4-fluorophényl)pipérazin-1-yl]-3-[2-oxypipéridin-1-yl]propane,
(j) 1-[4-(2-éthylphényl)pipérazin-1-yl]-3-[2-oxypipéridin-1-yl]propane,
(k) 1-[4-(2-méthoxyphényl)pipérazin-1-yl]-3-[2-oxypipéridin-1-yl]propane,
(l) 1-[4-(2-pyridyl)pipérazin-1-yl]-3-[2-oxypipéridin-1-yl]propane,
(m) 1-[4-(3-trifluorométhylphényl)pipérazin-1-yl]-3-[2-oxypipéridin-1-yl]propane.

3. Procédé selon la revendication 1, dans lequel le rapport molaire entre les composés de formule 2 et 5 est 1/1.

4. Procédé selon la revendication 1, dans lequel le solvant utilisé est sélectionné parmi le toluène ou le xylène et la quantité du solvant utilisé va de 5 à 6 ml par mmole des composés réactionnels de formule 2 et 5.

5. Procédé selon la revendication 1, dans lequel le rapport atomique/molaire entre la base métal de sodium/potassium ou butoxyde tertiaire de potassium et les composés de formule 2 et 5 est 1/1.

6. Composition pharmaceutique comprenant un composé de formule 1 tel que défini à la revendication 1 en mélange avec un support classique pharmaceutiquement acceptable.

7. Procédé pour la préparation d'une composition pharmaceutique qui comprend le fait d'amener un composé de la formule 1 tel que défini à la revendication 1 en association avec un support pharmaceutiquement acceptable.

8. Utilisation d'un composé de formule 1 tel que défini à la revendication 1 dans la fabrication d'un médicament pour le traitement de l'un quelconque parmi les troubles suivants : l'hypertension chez les mammifères, les maladies vasculaires périphériques chez les mammifères, les maladies provenant de modifications/détérioration des circulations centrale/périphérique et des systèmes de récepteurs adrénergiques, telles que l'ischémie myocardique, l'infarctus du myocarde (IM), l'angine de poitrine, toute intervention chirurgicale cardiaque, l'ischémie rénale, l'insuffisance circulatoire des extrémités, l'accident vasculaire cérébral et le trauma, la blessure de reperfusion chez les mammifères, les maladies ischémiques comme l'infarctus du myocarde (IM) ou l'angine de poitrine.

9. Composé de formule I 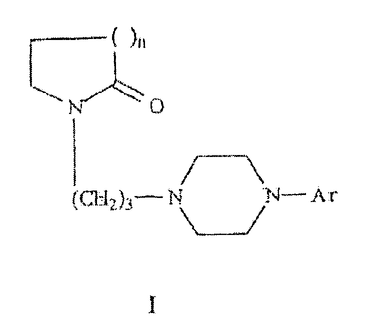 dans laquelle Ar représente pyridyle ou phényle, ou un groupe phényle substitué par un ou plusieurs groupes alkyle, alcoxy ou halogène, ou par un ou plusieurs groupes CF₃, n = 1 ou n = 2, y compris les composés :
(a) 1-[4-(3-chlorophényl)pipérazin-1-yl]-3-[2-oxypyrrolidin-1-yl]propane,
(b) 1-[4-(4-chlorophényl)pipérazin-1-yl]-3-[2-oxypyrrolidin-1-yl]propane,
(c) 1-[4-(3-fluorophényl)pipérazin-1-yl]-3-[2-oxypyrrolidin-1-yl]propane,
(d) 1-[4-(4-fluorophényl)pipérazin-1-yl]-3-[2-oxypyrrolidin-1-yl]propane,
(e) 1-[4-(4-éthylphényl)pipérazin-1-yl]-3-[2-oxypyrrolidin-1-yl]propane,
(f) 1-[4-(2-éthylphényl)pipérazin-1-yl]-3-[2-oxypyrrolidin-1-yl]propane,
(g) 1-[4-(4-chlorophényl)pipérazin-1-yl]-3-[2-oxypipéridin-1-yl]propane,
(h) 1-[4-(3-chlorophényl)pipérazin-1-yl]-3-[2-oxypipéridin-1-yl]propane,
(i) 1-[4-(4-fluorophényl)pipérazin-1-yl]-3-[2-oxypipéridin-1-yl]propane,
(j) 1-[4-(2-éthylphényl)pipérazin-1-yl]-3-[2-oxypipéridin-1-yl]propane,
(k) 1-[4-(2-méthoxyphényl)pipérazin-1-yl]-3-[2-oxypipéridin-1-yl]propane,
(l) 1-[4-(2-pyridyl)pipérazin-1-yl]-3-[2-oxypipéridin-1-yl]propane,
(m) 1-[4-(3-trifluorométhylphényl)pipérazin-1-yl]-3-[2-oxypipéridin-1-yl]propane.

10. Composé selon la revendication 9 destiné à être utilisé comme médicament.
